# FASCICULE DE BREVET EUROPEEN

(11) **EP 1 497 274 B1**
(45) Date de publication et mention de la délivrance du brevet: **13.09.2006**
(21) Numéro de dépôt: 03745842.9
(22) Date de dépôt: 10.04.2003
(51) Int. Cl.: C07D 295/22, C07C 233/65, C07C 69/76, C07C 63/72, C07C 63/331, C07C 65/24, A61K 31/165, A61K 31/445, A61P 25/00

(54) **DERIVES DE TERPHENYLE, LEUR PREPARATION, LES COMPOSITIONS PHARMACEUTIQUES EN CONTENANT**
TERPHENYLDERIVATE, DEREN HERSTELLUNG UND ZUSAMMENSETZUNGEN, DIE DIESE ENTHALTEN
TERPHENYL DERIVATIVES, PREPARATION THEREOF, COMPOSITIONS CONTAINING SAME

(30) Priorité: 11.04.2002 FR 0204567
(43) Date de publication de la demande: 19.01.2005
(73) Titulaire: Sanofi-Aventis, 75013 Paris (FR)
(72) Inventeur: BARTH, Francis, F-34680 Saint-Georges d'Orques (FR); MARTINEZ, Serge, F-34000 Montpellier (FR); RINALDI-CARMONA, Murielle, F-34680 Saint-Georges d'Orques (FR)
(74) Mandataire: Tsitini-Souleau, Maria
(86) Numéro de dépôt international: PCT/FR2003/001134
(87) Numéro de publication internationale: WO 2003/084943

(56) Documents cités:
- FR-A- 2 800 375
- US-A- 4 916 145
- RINALDI-CARMONA M ET AL: "SR141716A, A POTENT AND SELECTIVE ANTOGONIST OF THE BRAIN CANNABINOID RECEPTOR" FEBS LETTERS, ELSEVIER SCIENCE PUBLISHERS, AMSTERDAM, NL, vol. 350, no. 2/3, 1994, pages 240-244, XP002044764 ISSN: 0014-5793 cité dans la demande
- BRENNA, ELISABETTA ET AL: "New route to o-terphenyls: application to the synthesis of 6,7,10,11-tetramethoxy-2-(methoxycarbonyl) triphenylene" JOURNAL OF THE CHEMICAL SOCIETY, PERKIN TRANSACTIONS 1: ORGANIC AND BIO-ORGANIC CHEMISTRY (1998), (5), 901-904, XP002221111
- SATO, TAKEO ET AL: "Photoaryl coupling reactions. VII. New route to polycondensed aromatics Photolytic formation of triphenylene and dibenzo[fg, op]naphthacene ring systems" BULL. CHEM. SOC. JAP. (1971), 44(9), 2484-90, XP002221112
- BASS, CAROLINE E. ET AL: "SR-141716A-induced stimulation of locomotor activity. A structure-activit relationship study" PHARMACOLOGY, BIOCHEMISTRY AND BEHAVIOR (2002), 74(1), 31-40, XP002256669

## Description

La présente invention se rapporte à des dérivés de terphényle, à leur préparation et à des compositions pharmaceutiques les contenant. La demande de brevet FR 2 800 375 décrit des dérivés tricycliques d'acide pyrazole carboxylique qui sont actifs sur les récepteurs aux cannabinoïdes CBᵢ.

Ainsi, la présente invention a pour objet des composés de formule : 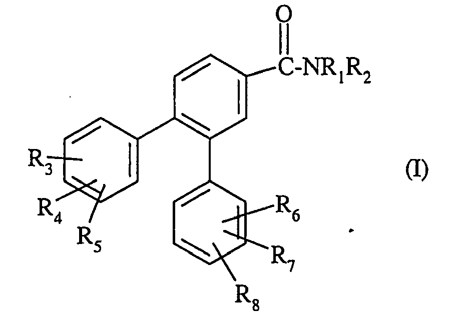 dans laquelle :
- R₁ représente l'hydrogène ou un (C₁-C₄)alkyle ;
- R₂ représente : . un groupe (C₃-C₇)alkyle,
   . un groupe indan-1-yle ou 1,2,3,4-tétrahydronaphtalèn-1-yle, lesdits groupes étant non substitués ou substitués par un atome d'halogène et/ou un groupe méthyle ;
   . un radical hétérocyclique monoazoté, saturé, de 5 à 7 atomes, l'atome d'azote étant substitué par un groupe (C₁-C₄)alkyle, benzyle, (C₁-C₃)alcoxycarbonyle ou (C₁-C₄)alcanoyle ;
   . un groupe NR₉R₁₀ ;
   . un groupe (CH₂)ₙR₁₁, CH(CH₃)R₁₁, (CH₂)ₘN(CH₃)R₁₁ ;
   . un radical carbocyclique non aromatique en C₃-C₁₂, non substitué ou substitué une ou plusieurs fois par un groupe méthyle ;
- ou R₁ et R₂ ensemble avec l'atome d'azote auquel ils sont liés constituent soit un radical pipérazin-1-yle substitué en -4 par un groupe phényle ou benzyle, soit un radical pipéridin-1-yle disubstitué en -4 par un groupe phényle ou benzyle et par un groupe (C₁-C₄)alkyle ou (C₁-C₃)alcanoyle ; les groupes phényles ou benzyles substituants le radical pipérazin-1-yle ou le radical pipéridin-1-yle étant non substitués ou substitués par un atome d'halogène et/ou un groupe méthyle ;
- R₃, R₄, R₅, R₆, R₇, R₈ représentent chacun indépendamment l'un de l'autre un atome d'hydrogène ou d'halogène, un groupe (C₁-C₆)alkyle, (C₁-C₆)alcoxy ou trifluorométhyle ;
- R₉ et R₁₀ ensemble avec l'atome d'azote auquel ils sont liés constituent un radical hétérocyclique, saturé ou insaturé, de 5 à 10 atomes, contenant ou non un deuxième hétéroatome choisi parmi O ou N, ledit radical étant non substitué ou substitué une ou plusieurs fois par un groupe (C₁-C₄)alkyle, hydroxyle, ou (C₁₋C₄)alcoxy ;
- R₁₁ représente :
   . un phényle non substitué ou substitué par un ou plusieurs substituants choisi parmi un atome d'halogène ou un groupe méthyle ;
   . un radical hétéroaryle de 6 à 10 atomes contenant un ou plusieurs atomes d'azote ;
- n représente 1, 2 ou 3 ;
- m représente 0, 2 ou 3 ;
ainsi que leurs sels, leurs solvats et leurs hydrates.

Les composés de formule (I) peuvent exister à l'état de bases ou de sels d'addition à des acides. Ces sels sont avantageusement préparés avec des acides pharmaceutiquement acceptables, mais les sels d'autres acides utiles, par exemple, pour la purification ou l'isolement des composés de formule (I) font également partie de l'invention.

Par groupe alkyle, on entend un radical linéaire ou ramifié, tel que, en particulier : méthyle, éthyle, propyle, isopropyle, butyle, isobutyle, *tert*-butyle, n-pentyle, isopentyle, n-hexyle, isohexyle, le groupe méthyle étant préféré pour un (C₁₋C₄)alkyle, les groupes *tert*-butyle, 2-méthylbutyl-2, 3,3-diméthylbutyl-2, étant préféré pour un (C₁-C₆)alkyle.

Par groupe (C₁-C₆)alcoxy, on entend un radical linéaire ou ramifié contenant 1 à 6 atomes de carbone, le groupe méthoxy étant préféré.

Par atome d'halogène, on entend un atome de fluor, de chlore, de brome ou d'iode; les atomes de fluor, chlore ou brome étant préférés.

Les radicaux carbocycliques non aromatiques en C₃-C₁₂ comprennent les radicaux mono ou polycycliques, condensés ou pontés. Les radicaux monocycliques incluent les cycloalkyles par exemple cyclopropyle, cyclobutyle, cyclopentyle, cyclohexyle, cycloheptyle, cyclooctyle ; le cyclohexyle et le cyclopentyle étant préférés. Les radicaux di- ou tricycliques condensés, pontés ou spiraniques, incluent par exemple les radicaux norbornyle, bomyle, isobomyle, noradamantyle, adamantyle, spiro[5.5]undécanyle, bicyclo[2.2.1]heptanyle ; le spiro[5.5]undécanyle et le bicyclo[2.2.1]heptanyle étant préférés.

Par radical hétérocyclique, saturé ou insaturé, de 5 à 10 atomes, contenant ou non un deuxième hétéroatome tel que O ou N, on entend des radicaux tel que morpholin-4-yle, pipéridin-1-yle, pipérazin-1-yle, pyrrolidin-1-yle, 3,6-dihydropyridin-1-yle, octahydrocyclopenta[c]pyrrol-2-yle, les radicaux pyrrolidin-1-yle, pipéridin-1-yle et morpholin-4-yle étant préférés.

Parmi les composés selon l'invention, on préfère les composés de formule (I) danq laquelle :
- R₁ représente un atome d'hydrogène ou un groupe (C₁-C₄)alkyle ;
- R₂ représente un groupe NR₉R₁₀ ou un radical carbocyclique non aromatique en C₃-C₁₂, non substitué ou substitué une ou plusieurs fois par un groupe méthyle ;
- R₃, R₄, R₅, R₆, R₇ et R₈ représentent chacun indépendamment l'un de l'autre un atome d'hydrogène ou d'halogène, un groupe (C₁-C₆)alkyle, (C₁-C₆)alcoxy ou trifluorométhyle ;
- R₉ et R₁₀ ensemble avec l'atome d'azote auquel ils sont liés constituent un radical hétérocyclique, saturé ou insaturé, de 5 à 10 atomes, contenant ou non un deuxième hétéroatome choisi parmi O ou N, ledit radical étant non substitué ou substitué une ou plusieurs fois par un groupe (C₁-C₆)alkyle ;
ainsi que leurs sels, leur solvats et leurs hydrates.

Parmi les composés objets de l'invention, on peut citer les composés préférés qui se définissent par les valeurs suivantes des substituants :
- R₁ représente un atome d'hydrogène ; et/ou
- R₂ représente un groupe choisi parmi pipéridin-1-yle, pyrrolidin-1-yle, cyclohexyle, spiro[5.5]undécanyle, 1,3,3-triméthylbicyclo[2.2.1]heptan-2-yle ; et/ou
- au moins l'un des substitutants R₃, R₄, R₅ représente un atome d'halogène ou un groupe trifluorométhyle ; et/ou
- au moins l'un des substituants R₆, R₇, R₈ représente un atome d'halogène.

La présente invention a également pour objet un procédé de préparation des composés de formule (I). Ce procédé est caractérisé en ce qu'on traite un dérivé fonctionnel de l'acide terphénylique de formule : 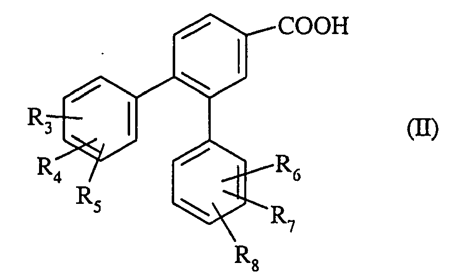 dans laquelle R₃, R₄, R₅, R₆, R₇, R₈ sont tels que définis pour (I) avec une amine de formule HNR₁R₂ (III) dans laquelle R₁ et R₂ sont tels que définis pour (I). Eventuellement, on transforme le composé ainsi obtenu en l'un de ses sels et/ou solvats.

Comme dérivé fonctionnel de l'acide (II) on peut utiliser le chlorure d'acide, l'anhydride, un anhydride mixte, un ester alkylique en C₁-C₄ dans lequel l'alkyle est droit ou ramifié, un ester activé, par exemple l'ester de *p*-nitrophényle ou l'acide libre opportunément activé, par exemple, avec le N,N-dicyclohexylcarbodiimide ou avec l'hexafluorophosphate de benzotriazol-1-yloxytris(diméthylamino)phosphonium (BOP).

Ainsi dans le procédé selon l'invention, on peut faire réagir le chlorure de l'acide pyrazole-3-carboxylique, obtenu par réaction du chlorure de thionyle sur l'acide de formule (II), avec une amine HNR₁R₂, dans un solvant inerte tel qu'un solvant chloré (le dichlorométhane, le dichloroéthane, le chloroforme par exemple), un éther (tétrahydrofurane, dioxane par exemple) ou un amide (N,N-diméthylformamide par exemple) sous une atmosphère inerte, à une température comprise entre 0°C et la température ambiante, en présence d'une amine tertiaire telle que la triéthylamine, la N-méthylmorpholine ou la pyridine.

Une variante consiste à préparer l'anhydride mixte de l'acide de formule (II) par réaction du chloroformiate d'éthyle avec l'acide de formule (II), en présence d'une base telle que la triéthylamine, et à le faire réagir avec une amine HNR₁R₂, dans un solvant tel que le dichlorométhane, sous une atmosphère inerte, à la température ambiante, en présence d'une base telle que la triéthylamine.

Les acides de formule (II) peuvent être préparés selon le schéma ci-après :

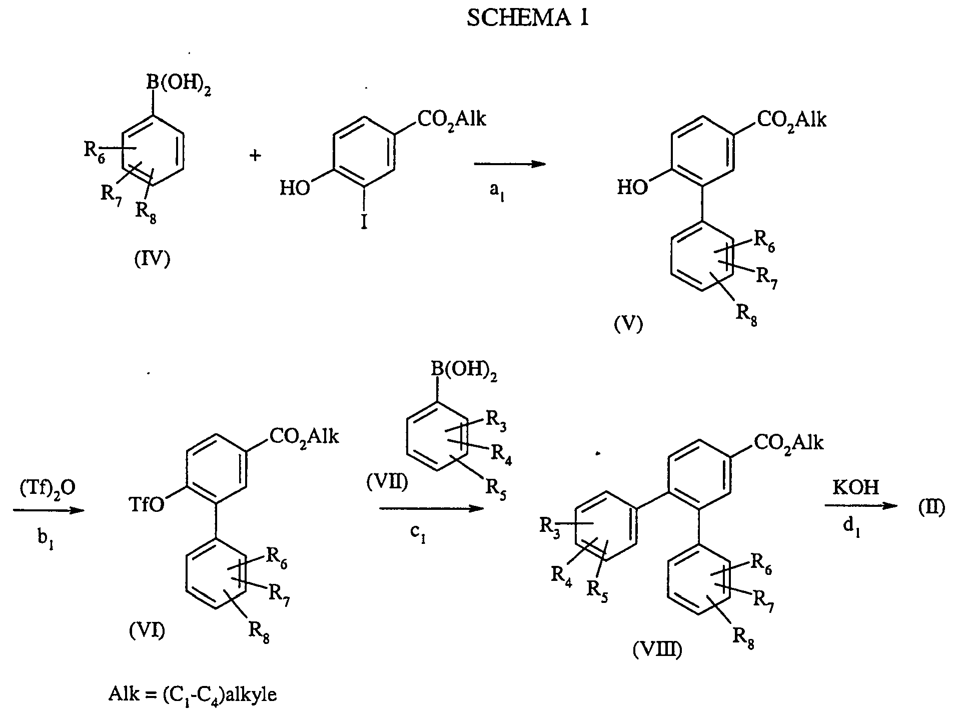

A l'étape a₁, la réaction de l'organoborate de formule (IV) avec un ester de l'acide 4-hydroxy-3-iodobenzoïque est effectuée selon Farmaco Ed. Sci., 1958, 13, 121 en utilisant les conditions décrites par Suzuki dans Helv. Chim. Acta, 1992, 75, 855.

A l'étape b₁, on fait agir l'anhydride triflique ((Tf)₂O) dans la pyridine pour préparer le composé de formule (VI). Ce composé est couplé, à l'étape c₁ avec un organoborate de formule (VII) selon les conditions décrites dans J. Org. Chem., 1992, 57, 379.

L'ester de terphényle ainsi formé est ensuite saponifié par des méthodes connues, par exemple en présence de potasse, pour conduire à l'acide de formule (II).

Les composés de formule (II) dans laquelle tous les substituants R₃ à R₈ sont l'hydrogène sont décrits dans le brevet US 4,916,145 et dans la publication de T. Sato et al., Bull. Chem. Soc. Jap., 1971, 44(9), 2484-2490 ; les composés de formule (II) dans laquelle les substituants R₃ et R₆ sont simultanément un 3-methoxy, un 4-methoxy ou un 3-fluor, les autres substituants R₄, R₅, R₆, R₇ et R₈ étant l'hydrogène sont décrits dans le brevet US 4,916,145 ; les composés de formule (II) dans laquelle les substituants R₃, R₄ et R₆, R₇ sont simultanément 3,4-diméhoxy, les substituants R₅ et R₈ étant l'hydrogène sont décrits dans la publication de E. Brenna, J. Chem. Soc. Perkin Trans. I, 1998, 901-904.

Les autres acides de formule (II) et leurs esters de formule (VIII) sont nouveaux et constituent un aspect ultérieur de l'invention. Ainsi, la présente invention a également pour objet les composés de formule : 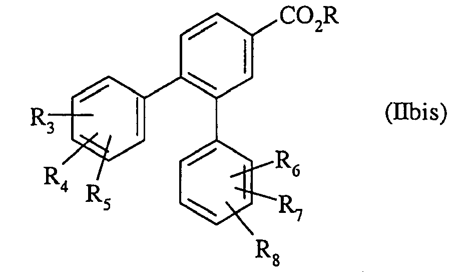 dans laquelle R₃, R₄, R₅, R₆, R₇, R₈ sont tels que définis pour (I) et R représente un atome d'hydrogène ou un groupe (C₁-C₄)alkyle à la condition que R₃, R₄, R₅, R₆, R₇, R₈ ne soient pas simultanément l'hydrogène, et à la condition que lorsque R₄, R₅, R₇ et R₈ représentent l'hydrogène, R₃ et R₆ ne représentent pas simultanément un atome de fluor en méta, ni un groupe méthoxy en méta ou en para, et à la condition que lorsque R₅ et R₈ représentent l'hydrogène, R₃, R₄ et R₅, R₆ ne représentent pas simultanément les groupes 3,4-diméthoxy.

Plus particulièrement, on préfère les composés de formule (IIbis) dans laquelle :
- R₃ est en position -4 et représente un atome d'halogène ou un groupe trifluorométhyle ;
- R₆ est en position -2 et représente un atome d'hydrogène ou d'halogène ;
- R₇ est en position -4 et représente un atome d'halogène ;
- R₄, R₅ et R₈ sont l'hydrogène.

Les amines HNR₁R₂ (III) sont connues ou préparées par des méthodes connues, on peut citer par exemple : Chem. Ber., 1986, 119, 1413-1423.

Les composés de formule (I) possèdent une très bonne affinité *in vitro* (IC₅₀ ≤ 10⁻⁷M) pour les récepteurs aux cannabinoïdes CB₁, dans les conditions expérimentales décrites par M. Rinaldi-Carmona et al. (FEBS Letters, 1994, 350, 240-244).

La nature antagoniste des composés de formule (I) a été démontrée par les résultats obtenus dans les modèles de l'inhibition de l'adénylate-cyclase comme décrits dans M. Rinaldi-Carmona et al., J. Pharmacol. Exp. Ther., 1996, 278, 871-878.

La toxicité des composés de formule (I) est compatible avec leur utilisation en tant que médicament.

Selon un autre de ses aspects, la présente invention concerne l'utilisation d'un composé de formule (I), ou de l'un de ses sels, solvats ou hydrates pharmaceutiquement acceptables, pour la préparation de médicaments destinés à traiter les maladies impliquant les récepteurs aux cannabinoïdes CB₁.

Par exemple et de manière non limitative, les composés de formule (I) sont utiles comme médicaments psychotropes, notamment pour le traitement des désordres psychiatriques incluant l'anxiété, la dépression, les troubles de l'humeur, l'insomnie, les troubles délirants, les troubles obsessionnels, les psychoses en général, la schizophrénie, ainsi que pour le traitement des troubles liés à l'utilisation de substances psychotropes, notamment dans le cas d'un abus d'une substance et/ou de dépendance à une substance, y compris la dépendance alcoolique et la dépendance nicotinique.

Les composés de formule (I) selon l'invention peuvent être utilisés comme médicaments pour le traitement de la migraine, du stress, des maladies d'origine psychosomatique, des crises d'attaques de panique, de l'épilepsie, des troubles du mouvement, en particulier des dyskinésies ou de la maladie de Parkinson, des tremblements et de la dystonie.

Les composés de formule (I) selon l'invention peuvent également être utilisés comme médicaments dans le traitement des troubles mnésiques, des troubles cognitifs, en particulier dans le traitement des démences séniles, de la maladie d'Alzheimer, ainsi que dans le traitement des troubles de l'attention ou de la vigilance. De plus, les composés de formule (I) peuvent être utiles comme neuroprotecteurs, dans le traitement de l'ischémie, des traumatismes crâniens et le traitement des maladies neurodégénératives : incluant la chorée, la chorée de Huntington, le syndrome de Tourrette.

Les composés de formule (I) selon l'invention peuvent être utilisés comme médicaments dans le traitement de la douleur : les douleurs neuropathiques, les douleurs aiguës périphériques, les douleurs chroniques d'origine inflammatoire.

Les composés de formule (I) selon l'invention peuvent être utilisés comme médicaments dans le traitement des troubles de l'appétit, de l'appétence (pour les sucres, carbohydrates, drogues, alcools ou toute substance appétissante) et/ou des conduites alimentaires, notamment en tant qu'anorexigènes ou pour le traitement de l'obésité ou de la boulimie ainsi que pour le traitement du diabète de type II ou diabète non insulinodépendant. De plus, les composés de formule (I) selon l'invention peuvent être utilisés en tant que médicaments dans le traitement des troubles gastro-intestinaux, des troubles diarrhéiques, des ulcères, des vomissements, des troubles vésicaux et urinaires, des troubles d'origine endocrinienne, des troubles cardio-vasculaires, de l'hypotension, du choc hémorragique, du choc septique, de la cirrhose chronique du foie, de l'asthme, du syndrome de Raynaud, du glaucome, des troubles de la fertilité, des phénomènes inflammatoires, des maladies du système immunitaire, en particulier autoimmunes et neuroinflammatoires tel que l'arthrite rhumatoïde, l'arthrite réactionnelle, les maladies entraînant une démyélinisation, la sclérose en plaque, des maladies infectieuses et virales telles que les encéphalites, des accidents vasculaires cérébraux ainsi qu'en tant que médicaments pour la chimiothérapie anticancéreuse et pour le traitement du syndrome de Guillain-Barré.

Selon la présente invention, les composés de formule (I) sont tout particulièrement utiles pour le traitement des troubles psychotiques, en particulier la schizophrénie ; pour le traitement des troubles de l'appétit et de l'obésité pour le traitement des troubles mnésiques et cognitifs ; pour le traitement de la dépendance alcoolique, de la dépendance nicotinique, c'est à dire pour le sevrage alcoolique et pour le sevrage tabagique.

Selon un de ses aspects, la présente invention est relative à l'utilisation d'un composé de formule (I) de ses sels pharmaceutiquement acceptables et de leurs solvats ou hydrates pour le traitement des troubles et maladies indiqués ci-dessus.

Le composé selon l'invention est généralement administré en unité de dosage.

Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (I), un de ses sels pharmaceutiquement acceptables ou un de leurs solvats.

Le composé de formule (I) ci-dessus et ses sels ou solvats pharmaceutiquement acceptables peuvent être utilisés à des doses journalières de 0,01 à 100 mg par kg de poids corporel du mammifère à traiter, de préférence à des doses journalières de 0,02 à 50 mg/kg. Chez l'être humain, la dose peut varier de préférence de 0,05 à 4000 mg par jour, plus particulièrement de 0,1 à 1000 mg par jour selon l'âge du sujet à traiter ou le type de traitement, à savoir prophylactique ou curatif. Bien que ces dosages soient des exemples de situations moyennes, il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, l'âge, le poids et la réponse dudit patient.

Dans les compositions pharmaceutiques de la présente invention pour l'administration orale, sublinguale, inhalée, sous-cutanée, intramusculaire, intraveineuse, transdermique, locale ou rectale, le principe actif peut être administré sous forme unitaire d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les aérosols, les formes d'administration topique, les implants, les formes d'administration sous-cutanée, intramusculaire, intraveineuse, intranasale ou intra-oculaire et les formes d'administration rectale.

Dans les compositions pharmaceutiques de la présente invention, le principe actif est généralement formulé en unités de dosage contenant de 0,05 à 1000 mg, avantageusement de 0,1 à 500 mg, de préférence de 1 à 200 mg dudit principe actif par unité de dosage pour les administrations quotidiennes.

Dans la présente description, on utilise les abréviations suivantes :
DCM : dichlorométhane
DMF : diméthylformamide
AcOEt : acétate d'éthyle
TA : température ambiante
F : point de fusion.

Les composés selon l'invention sont analysés par couplage LC/UV/MS (chromatographie liquide/détection UV/spectrométrie de masse). On mesure le pic moléculaire (M⁺) et le temps de rétention (t) en minutes.

On utilise une colonne Xterra Waters® MS C18, commercialisée par Waters, de 2,1 x 30 mm, 3,5 µm, à température ambiante, débit 1 mL/minute.

L'éluant est composé comme suit :
- solvant A : 0,025 % d'acide trifluoroacétique (TFA) dans l'eau
- solvant B : 0,025 % de TFA dans l'acétonitrile.

Gradient : Le pourcentage de solvant B varie de 0 à 100 % en 2 minutes avec un plateau à 100 % de B pendant 1 minute.

La détection UV est effectuée entre 210 nm et 400 nm et la détection de masse en mode ionisation chimique à pression atmosphérique.

Pour l'interprétation des spectres de résonance magnétique nucléaire (RMN) les abréviations suivantes sont utilisées : s : singulet ; d : doublet ; m : massif : se : singulet élargi ; dd : doublet de doublet.

### Préparation 1.1

(II bis) : R₃, R₄, R₅ = 4-Cl ; R₆, R₇, R₈ = 2,4-diCl.

4,2",4"-trichloro[1,1';2',1"]terphényl-4'-carboxylate de méthyle.

### A) Acide 4-hydroxy-3-iodobenzoïque.

On place 30 g d'acide 4-hydroxybenzoïque dans 780 ml d'eau contenant 18 g de soude, on ajoute 49,5 g d'iodure de sodium puis on coule lentement 675 ml d'une solution d'hypochlorite de sodium à 3,5 % et on laisse 13 heures sous agitation à TA. On ajoute 60 ml d'H₂SO₄ concentré puis, après refroidissement, on filtre le précipité formé et on le rince à l'eau. On obtient 32,46 g du composé attendu, F = 163°C.

### B) 4-Hydroxy-3-iodobenzoate de méthyle.

On place 32,46 g de l'acide obtenu à l'étape précédente dans un mélange contenant 138 ml de méthanol et 10,36 ml d'acide sulfurique concentré et on chauffe à reflux pendant 3 heures et demie. On concentre sous vide le solvant, reprend le résidu par de l'eau déminéralisée et de l'éther éthylique. On neutralise par Na₂CO₃ puis on extrait la phase aqueuse par AcOEt. On lave à l'eau, puis par une solution saturée de NaCl. On obtient 32 g du composé attendu.

### C) 2',4'-Dichloro-6-hydroxy-(1,1'-biphényl)carboxylate de méthyle.

On introduit sous argon 5,6 g de 4-hydroxy-3-iodobenzoate de méthyle dans 50 ml de DMF anhydre puis 4,2 g d'acide 2,4-dichlorophényl boronique et 5,54 ml de triéthylamine puis on ajoute 240 mg de tri-*ortho*tolylphosphine et on laisse sous argon pendant 1 heure. On ajoute 180 mg d'acétate de palladium puis on chauffe 4heures à 100°C. On rajoute 2 g d'acide 2,4-dichloro-phénylboronique, 5,54 ml de triéthylamine, 120 mg de tri-*ortho*tolylphosphine et 180 mg d'acétate de palladium puis on chauffe 8 heures à 100°C. On concentre sous vide, reprend le résidu par AcOEt puis on lave par une solution de NH₄OH à 10 %. On extrait par AcOEt, lave à l'eau puis une solution saturée de NaCl. Le résidu est séché puis chromatographié sur silice en éluant par un mélange cyclohexane/AcOEt (82/18 ; v/v) pour obtenir 3,4 g du composé attendu.

### D) 2',4'-Dichloro-6-((trifluorométhylsulfonyl)oxy)(1,1'-biphényl)-3-carboxylate de méthyle.

On place 3,27 g du composé obtenu à l'étape précédente dans 150 ml de pyridine, on refroidit le milieu entre 0°C et 5°C et coule goutte à goutte 2,8 ml d'anhydride triflique. On maintient sous agitation à TA pendant une nuit puis on concentre à sec. Le résidu est chromatographié sur silice en éluant par un mélange cyclohexane/AcOEt (90/10 ; v/v) pour obtenir 3,2 g du composé attendu.

### E) 4,2",4"-trichloro[1,1' ; 2',1"]terphényl-4'-carboxylate de méthyle.

On place 3,2 g du composé obtenu à l'étape précédente dans 75 ml de toluène et on ajoute 2,33 g d'acide 4-chlorophénylboronique puis 1,55 g de carbonate de potassium. On laisse sous argon pendant 30 minutes puis on ajoute 1,38 g de tétrakis (triphénylphosphine) palladium et on chauffe le milieu réactionnel entre 80°C et 85°C pendant 3 heures. On laisse une nuit à TA puis on dilue par AcOEt, lave par une solution de Na₂CO₃ à 5 % (2 fois), puis par une solution saturée de NaCl. On sèche, puis le résidu est chromatographié sur silice par un mélange cyclohexane/AcOEt (80/20 ; v/v) pour donner 1,83 g du composé attendu qui cristallise dans l'éther isopropylique, F = 136°C.

En procédant comme décrit ci-dessus, on prépare les esters méthyliques des acides de formule (II) rassemblés dans le tableau ci-après.

**TABLEAU 1**

| | | | |
|---|---|---|---|
| 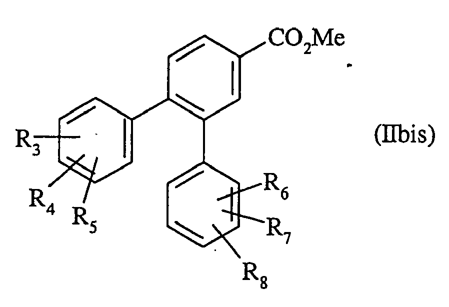 | | | |

| Préparations | R₃, R₄, R₅ | R₆, R₇, R₈ | F°C/RMN |
|---|---|---|---|
| 1.2 | 4-Cl | 4-Cl | 223°C |
| 1.3 | 4-F | 2,4-diCl | RMN (DMSO-d₆) δ ppm : 6,9 : m : 4H ; 7,25 : d : 1H ; 7,35 : dd : 1H ; 7,55 : m : 2H ; 7,80 : d : 1H ; 8,00 : dd : 1H ; 13,20 : se : 1H |
| 1.4 | 4-CF₃ | 2,4-diCl | 206°C |

### EXEMPLE 1 : Composé 1

4,2",4"-Trichloro-(N-1-pipéridinyl)-[1,1' ; 2',1"]terphényl-4'-carboxamide.

(I): R₁ =H ;

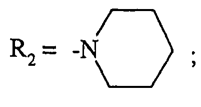

R₃, R₄, R₅ = 4-Cl ; R₆, R₇, R₈ = 2,4-diCl

### A) Acide 4,2",4"-trichloro[1,1' ; 2',1"]terphényl-4'-carboxylique.

On place 1,33 g du composé de la Préparation 1.1 en suspension dans 30 ml d'éthanol, on ajoute 0,95 g de potasse en solution dans 5 ml d'eau et on chauffe à reflux pendant 2 heures. Après refroidissement à TA, on filtre sur Célite® et on concentre à sec sous vide. Le résidu est repris par 30 ml d'eau puis acidifié à pH = 1 par addition d'HCl 1N. On refroidit le milieu à l'aide d'un bain de glace puis on extrait par AcOEt. On lave à l'eau puis par une solution saturée de NaCl pour obtenir 1,22 g du composé attendu, F = 237°C.

### B) Chlorure de l'acide 4,2",4"-trichloro[1,1' ; 2',1"]terphényl-4'-carboxylique.

On place 500 mg de l'acide obtenu à l'étape précédente, en suspension dans 50 ml de toluène, on ajoute 0,3 ml de chlorure de thionyle et on chauffe à reflux pendant 2 heures. On concentre à 2 reprises le solvant pour obtenir 0,52 g du composé attendu sous forme solide.

### C) 4,2",4"-Trichloro-(N-1-pipéridinyl)-[1,1' ; 2',1"]terphényl-4'-carboxamide.

On prépare une solution contenant 0,17 ml d'aminopipéridine et 0,22 ml de triéthylamine dans 10 ml de DCM, on refroidit cette solution entre 0°C et 5°C et l'on ajoute goutte à goutte, 0,52 g du chlorure d'acide obtenu à l'étape précédente dans 10 ml de DCM. On laisse 2 jours à + 4°C. On coule sur de l'eau glacée puis on extrait au DCM et lave par une solution de Na₂CO₃ à 5 % puis une solution saturée de NaCl. On sèche puis on chromatographie le résidu sur silice en éluant par un mélange toluène/AcOEt (88/12 ; v/v). On obtient 0,3 g du composé attendu, F = 182°C.

En procédant selon le mode opératoire de l'Exemple 1, on prépare les composés de l'invention décrits ci-après.

**TABLEAU 2**

| | | | | | |
|---|---|---|---|---|---|
| 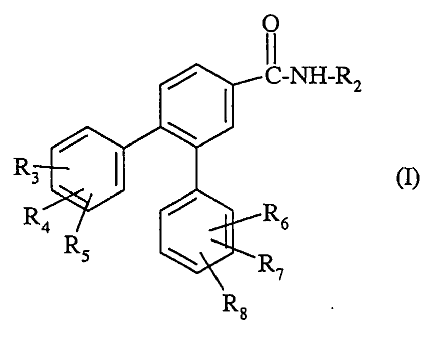 | | | | | |

| Composés | R₁ | R₂ | R₃, R₄, R₅ | R₆, R₇, R₈ | Caractérisation |
|---|---|---|---|---|---|
| 1 | H | 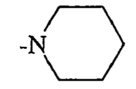 | 4-Cl | 2,4-diCl | F = 182°C |
| 2 | H | 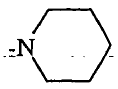 | 4-Cl | 4-Cl | F = 233°C |
| 3 | H | 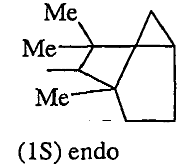 | 4-Cl | 2,4-diCl | F = 98°C |
| 4 | H | 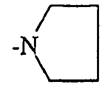 | 4-Cl | 2,4-diCl | F = 168°C |
| 5 | H | 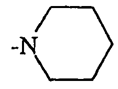 | 4-F | 2,4-diCl | F = 175°C |
| 6 | H | 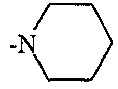 | 4-CF3 | 2,4-diCl | F = 177°C |
| 7 | H | 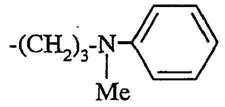 | 4-Cl | 4-Cl | M⁺ = 489,49 t = 1,95 |
| 8 | H | 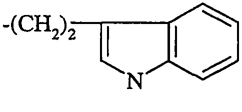 | 4-Cl | 4-Cl | M⁺ = 484,95 t = 2,33 |
| 9 | H | 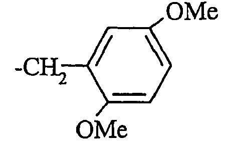 | 4-Cl | 4-Cl | M⁺ = 492,15 t = 2,28 |
| 10 | H | 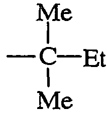 | 4-Cl | 4-Cl | M⁺= 411,98 t = 2,43 |
| 11 | H | 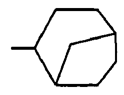 | 4-Cl | 4-Cl | M⁺ = 450,50 t = 2,43 |
| 12 | -NR₁R₂ | 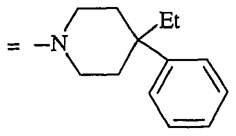 | 4-Cl | 4-Cl | M⁺ = 514,42 t = 2,50 |

## Revendications

1. Composés de formule : 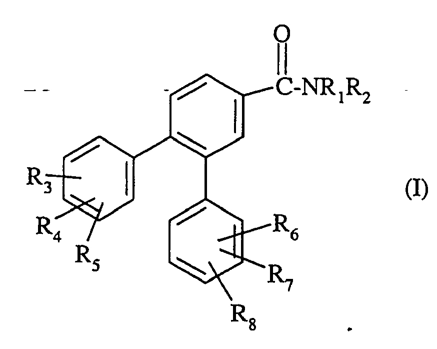 dans laquelle :
- R₁ représente l'hydrogène ou un (C₁-C₄)alkyle ;
- R₂ représente : . un groupe (C₃-C₇)alkyle ,
. un groupe indan-1-yle ou 1,2,3,4-tétrahydronaphtalèn-1-yle, lesdits groupes étant non substitués ou substitués par un atome d'halogène et/ou un groupe méthyle ;
. un radical hétérocyclique monoazoté, saturé, de 5 à 7 atomes, l'atome d'azote étant substitué par un groupe (C₁-C₄)alkyle, benzyle, (C₁-C₃)alcoxycarbonyle ou (C₁-C₄)alcanoyle ;
. un groupe NR₉R₁₀ ;
. un groupe (CH₂)ₙR₁₁, CH(CH₃)R₁₁, (CH₂)ₘN(CH₃)R₁₁ ;
. un radical carbocyclique non aromatique en C₃-C₁₂, non substitué ou substitué une ou plusieurs fois par un groupe méthyle ;
- ou R₁ et R₂ ensemble avec l'atome d'azote auquel ils sont liés constituent soit un radical pipérazin-1-yle substitué en -4 par un groupe phényle ou benzyle, soit un radical pipéridin-1-yle disubstitué en -4 par un groupe phényle ou benzyle et par un groupe (C₁-C₄)alkyle ou (C₁-C₃)alcanoyle ; les groupes phényles ou benzyles substituants le radical pipérazin-1-yle ou le radical pipéridin-1-yle étant non substitués ou substitués par un atome d'halogène et/ou un groupe méthyle ;
- R₃, R₄, R₅, R₆, R₇, R₈ représentent chacun indépendamment l'un de l'autre un atome d'hydrogène ou d'halogène, un groupe (C₁-C₆)alkyle, (C₁-C₆)alcoxy ou trifluorométhyle ;
- R₉ et R₁₀ ensemble avec l'atome d'azote auquel ils sont liés constituent un radical hétérocyclique, saturé ou insaturé, de 5 à 10 atomes, contenant ou non un deuxième hétéroatome choisi parmi O ou N, ledit radical étant non substitué ou substitué une ou plusieurs fois par un groupe (C₁-C₄)alkyle, hydroxyle, ou (C₁₋C₄)alcoxy ;
- R₁₁ représente : un phényle non substitué ou substitué par un ou plusieurs
substituants choisi parmi un atome d'halogène ou un groupe méthyle ;
. un radical hétéroaryle de 6 à 10 atomes contenant un ou plusieurs atomes d'azote ;
- n représente 1, 2 ou 3 ;
- m représente 0, 2 ou 3 ;
ainsi que leurs sels, leurs solvats et leurs hydrates.

2. Composé selon la revendication 1 de formule (I) dans laquelle :
- R₁ représente un atome d'hydrogène ou un groupe (C₁-C₄)alkyle ;
- R₂ représente un groupe NR₉R₁₀ ou un radical carbocyclique non aromatique en C₃-C₁₂, non substitué ou substitué une ou plusieurs fois par un groupe méthyle ;
- R₃, R₄, R₅, R₆, R₇ et R₈ représentent chacun indépendamment l'un de l'autre un atome d'hydrogène ou d'halogène, un groupe (C₁-C₆)alkyle, (C₁-C₆)alcoxy ou trifluorométhyle ;
- R₉ et R₁₀ ensemble avec l'atome d'azote auquel ils sont liés constituent un radical hétérocyclique, saturé ou insaturé, de 5 à 10 atomes, contenant ou non un deuxième hétéroatome choisi parmi O ou N, ledit radical étant non substitué ou substitué une ou plusieurs fois par un groupe (C₁-C₄)alkyle ; ainsi que leurs sels, leur solvats et leurs hydrates.

3. Composés selon la revendication 1 ou la revendication 2 de formule (I) dans laquelle :
- R₁ représente un atome d'hydrogène ; et/ou
- R₂ représente un groupe choisi parmi pipéridin-1-yle, pyrrolidin-1-yle, cyclohexyle, spiro[5.5]undécanyle, 1,3,3-triméthylbicyclo[2.2.1]heptan-2-yle ; et/ou
- au moins l'un des substituants R₃, R₄, R₅ représente un atome d'halogène ou un groupe trifluorométhyle ; et/ou
- au moins l'un des substituants R₆, R₇, R₈ représente un atome d'halogène.

4. Procédé de préparation d'un composé de formule (I) selon l'une quelconque des revendications 1 à 3 **caractérisé en ce qu'**on traite un dérivé fonctionnel de l'acide terphénylique de formule : 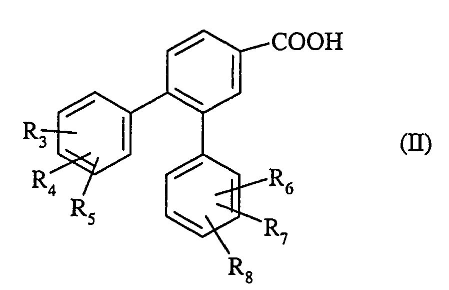 dans laquelle R₃, R₄, R₅, R₆, R₇, R₈ sont tels que définis pour un composé de formule (I) dans la revendication 1 avec une amine de formule HNR₁R₂ (III) dans laquelle R₁ et R₂ sont tels que définis pour un composé de formule (I) dans la revendication 1.

5. Composés de formule : 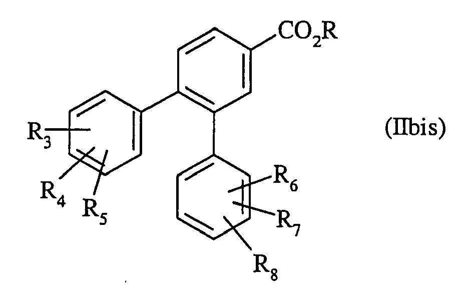 dans laquelle R₃, R₄, R₅, R₆, R₇, R₈ sont tels que définis pour un composé de formule (I) dans la revendication 1 et R représente un atome d'hydrogène ou un groupe (C₁-C₄)alkyle, à la condition que R₃, R₄, R₅, R₆, R₇, R₈ ne soient pas simultanément l'hydrogène, et à la condition que lorsque R₄, R₅, R₇ et R₈ représentent l'hydrogène, R₃ et R₆ ne représentent pas simultanément un atome de fluor en méta, ni un groupe méthoxy en méta ou en para, et à la condition que lorsque R₅ et R₈ représentent l'hydrogène, R₃, R₄ et R₅, R₆ ne représentent pas simultanément les groupes 3,4-diméthoxy.

6. Composé selon la revendication 5 de formule (IIbis) dans laquelle :
- R₃ est en position -4 et représente un atome d'halogène ou un groupe trifluorométhyle ;
- R₆ est en position -2 et représente un atome d'hydrogène ou d'halogène ;
- R₇ est en position -4 et représente un atome d'halogène ;
- R₄, R₅ et R₈ sont l'hydrogène.

7. Médicament **caractérisé en ce qu'**il comprend un composé de formule (I) selon l'une quelconque des revendications 1 à 3, ou un de ses sels, hydrates ou solvats pharmaceutiquement acceptables.

8. Composition pharmaceutique **caractérisée en ce qu'**elle comprend un composé de formule (I) selon l'une quelconque des revendications 1 à 3, ou un de ses sels, hydrates ou solvats pharmaceutiquement acceptables ainsi qu'au moins un excipient pharmaceutiquement acceptable.

9. Utilisation d'un composé de formule (I) selon l'une quelconque des revendications 1 à 3 pour la préparation d'un médicament destiné au traitement de toute maladie dans laquelle le récepteur aux cannabinoïdes CB₁ est impliqué.

10. Utilisation d'un composé de formule (I) selon l'une quelconque des revendications 1 à 3 pour la préparation d'un médicament destiné au traitement des troubles psychotiques, des troubles mnésifs et cognitifs, des troubles de l'appétit et de l'obésité, ou pour le sevrage tabagique ou le sevrage alcoolique.

## Claims

1. Compounds of formula: 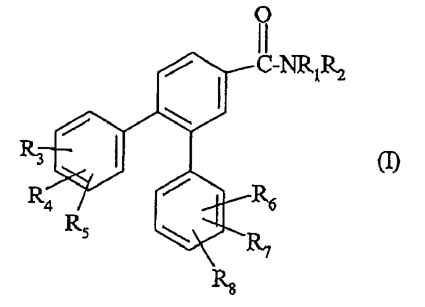 in which:
- R₁ represents hydrogen or a (C₁-C₄)alkyl;
- R₂ represents: . a (C₃-C₇)alkyl group,
. an indan-1-yl or 1,2,3,4-tetrahydronaphthalen-1-yl group, said groups being unsubstituted or substituted by a halogen atom and/or a methyl group;
. a saturated, single-nitrogen heterocyclic radical of 5 to 7 atoms, the nitrogen atom being substituted by a (C₁-C₄)alkyl, benzyl, (C₁-C₃) alkoxycarbonyl or (C₁-C₄)alkanoyl group;
. a group NR₉R₁₀;
. a group (CH₂)ₙR₁₁, CH(CH₃)R₁₁, (CH₂)ₘN(CH₃)R₁₁;
. a C₃-C₁₂ nonaromatic carbocyclic radical, unsubstituted or substituted one or more times by a methyl group;
- or R₁ and R₂ together with the nitrogen atom to which they are attached form either a piperazin-1-yl radical substituted in position 4 by a phenyl or benzyl group, or a piperidin-1-yl radical disubstituted in position 4 by a phenyl or benzyl group and by a (C₁-C₄)alkyl or (C₁-C₃)alkanoyl group; the phenyl or benzyl group substituents on the piperazin-1-yl radical or the piperidin-1-yl radical being unsubstituted or substituted by a halogen atom and/or a methyl group;
- R₃, R₄, R₅, R₆, R₇ and R₈ represent each independently of one another a hydrogen or halogen atom or a (C₁-C₆)alkyl, (C₁-C₆)alkoxy or trifluoromethyl group;
- R₉ and R₁₀ together with the nitrogen atom to which they are attached form a saturated or unsaturated heterocyclic radical of 5 to 10 atoms containing or not containing a second heteroatom selected from O and N, said radical being unsubstituted or substituted one or more times by a (C₁-C₄)alkyl, hydroxyl or (C₁-C₄)alkoxy group;
- R₁₁ represents: . a phenyl which is unsubstituted or substituted by one or more substituents selected from a halogen atom and a methyl group;
. a heteroaryl radical of 6 to 10 atoms containing one or more nitrogen atoms;
- n represents 1, 2 or 3;
- m represents 0, 2 or 3;
and their salts, their solvates and their hydrates.

2. Compounds according to Claim 1 of formula (I) in which:
- R₁ represents a hydrogen atom or a (C₁₋C₄)alkyl group;
- R₂ represents a group NR₉R₁₀ or a nonaromatic C₃-C₁₂ carbocyclic radical which is unsubstituted or substituted one or more times by a methyl group;
- R₃, R₄, R₅, R₆, R₇ and R₈ represent each independently of one another a hydrogen or halogen atom or a (C₁-C₆)alkyl, (C₁-C₆)alkoxy or trifluoromethyl group;
- R₉ and R₁₀ together with the nitrogen atom to which they are attached form a saturated or unsaturated heterocyclic radical of 5 to 10 atoms, containing or not containing a second heteroatom selected from O and N, said radical being unsubstituted or substituted one or more times by a (C₁-C₄)alkyl group;
and their salts, their solvates and their hydrates.

3. Compounds according to Claim 1 or Claim 2 of formula (I) in which:
- R₁ represents a hydrogen atom; and/or
- R₂ represents a group selected from piperidin-1-yl, pyrrolidin-1-yl, cyclohexyl, spiro[5.5]undecanyl and 1,3,3-trimethylbicyclo[2.2.1]heptan-2-yl; and/or
- at least one of the substituents R₃, R₄ and R₅ represents a halogen atom or a trifluoromethyl group; and/or
- at least one of the substituents R₆, R₇ and R₈ represents a halogen atom.

4. Process for preparing a compound of formula (I) according to any one of Claims 1 to 3, **characterized in that** a functional derivative of terphenylic acid of formula: 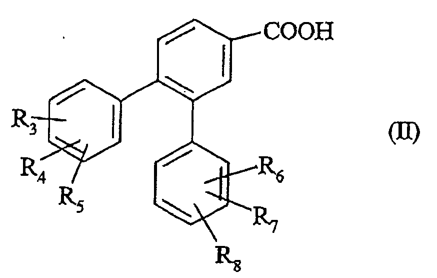 in which R₃, R₄, R₅, R₆, R₇ and R₈ are as defined for a compound of formula (I) in Claim 1 is treated with an amine of formula HNR₁R₂ (III) in which R₁ and R₂ are as defined for a compound of formula (I) in Claim 1.

5. Compounds of formula: 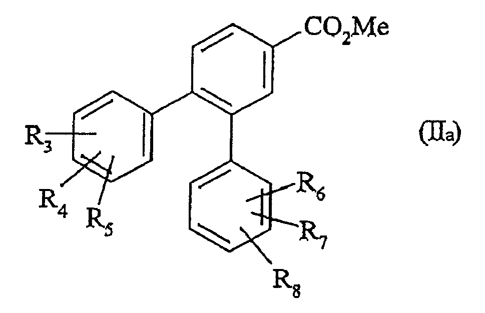 in which R₃, R₄, R₅, R₆, R₇ and R₈ are as defined for a compound of formula (I) in claim 1 and R represents a hydrogen atom or a (C₁-C₄)alkyl group, on condition that R₃, R₄, R₅, R₆, R₇ and R₈ are not simultaneously hydrogen, and on condition that, when R₄, R₅, R₇ and R₈ represent hydrogen, R₃ and R₆ do not simultaneously represent a fluorine atom in meta position, or a methoxy group in meta or para position, and on condition that when R₅ and R₈ represent hydrogen R₃, R₄ and R₅, R₆ do not simultaneously represent 3,4-dimethoxy groups.

6. Compounds according to Claim 5 of formula (IIa) in which:
- R₃ is in position 4 and represents a halogen atom or a trifluoromethyl group;
- R₆ is in position 2 and represents a hydrogen or halogen atom;
- R₇ is in position 4 and represents a halogen atom;
- R₄, R₅ and R₈ are hydrogen.

7. Medicinal product **characterized in that** it comprises a compound of formula (I) according to any one of Claims 1 to 3, or one of its pharmaceutically acceptable salts, hydrates or solvates.

8. Pharmaceutical composition **characterized in that** it comprises a compound of formula (I) according to any one of Claims 1 to 3, or one of its pharmaceutically acceptable salts, hydrates or solvates, and at least one pharmaceutically acceptable excipient.

9. Use of a compound of formula (I) according to any one of Claims 1 to 3 for preparing a medicinal product intended for treating any disease involving the CB₁ cannabinoid receptor.

10. Use of a compound of formula (I) according to any one of Claims 1 to 3 for preparing a medicinal product intended for treating psychotic disorders, memory and cognitive disorders, appetite disorders and obesity, or for tobacco withdrawal or alcohol withdrawal.

## Patentansprüche

1. Verbindungen der Formel: 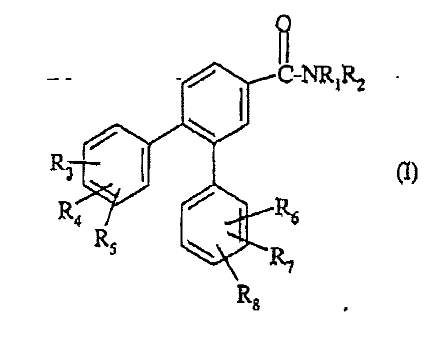 worin:
- R₁ für Wasserstoff oder eine C₁-C₄-Alkylgruppe steht;
- R₂ für .eine C₃-C₇-Alkylgruppe,
. eine Indan-1-yl- oder 1,2,3,4-Tetrahydronaphthalingruppe, wobei diese Gruppen gegebenenfalls durch ein Halogenatom und/oder eine Methylgruppe substituiert sind;
. einen gesättigten heterocyclischen Rest mit 5-7 Atomen und einem Stickstoffatom, welches durch eine C₁-C₄-Alkyl-, Benzyl-, C₁-C₃-Alkoxycarbonyl- oder C₁-C₄-Alkanoyl-gruppe substituiert ist;
. eine NR₉R₁₀-Gruppe;
. eine (CH₂)ₙR₁₁-, CH(CH₃)R₁₁- oder (CH₂)ₘN(CH₃)R₁₁-Gruppe;
. einen nichtaromatischen carbocyclischen C₃-C₁₂-Rest, der gegebenenfalls ein- oder mehrfach durch eine Methylgruppe substituiert ist;
steht;
- oder R₁ und R₂ gemeinsam mit dem Stickstoffatom, an das sie gebunden sind, einen in 4-Stellung durch eine Phenyl- oder Benzylgruppe substituierten Piperazin-1-ylrest oder einen in 4-Stellung durch eine Phenyl- oder Benzylgruppe und eine C₁-C₄-Alkyl- oder C₁-C₃-Alkanoylgruppe disubstituierten Piperidin-1-ylrest bilden, wobei die Phenyl- oder Benzylgruppensubstituenten an dem Piperazin-1-ylrest oder Piperidin-1-ylrest gegebenenfalls durch ein Halogenatom und/oder eine Methylgruppe substituiert sind;
- R₃, R₄, R₅, R₆, R₇ und R₈ jeweils unabhängig voneinander für ein Wasserstoff- oder Halogenatom, eine C₁-C₆-Alkyl-, C₁-C₆-Alkoxy- oder Trifluormethylgruppe stehen;
- R₉ und R₁₀ gemeinsam mit dem Stickstoffatom, an das sie gebunden sind, einen gesättigten oder ungesättigten heterocyclischen Rest mit 5 bis 10 Atomen bilden, der gegebenenfalls ein zweites unter O und N ausgewähltes Heteroatom enthält und gegebenenfalls ein- oder mehrfach durch eine C₁-C₄-Alkyl-, Hydroxyl- oder C₁-C₄₋Alkoxygruppe substituiert ist;
- R₁₁ für
. gegebenenfalls durch einen oder mehrere unter einem Halogenatom und einer Methylgruppe ausgewählte Substituenten substituiertes Phenyl
. einen Heteroarylrest mit 6 bis 10 Atomen und einem oder mehreren Stickstoffatomen
steht;
- n für 1, 2 oder 3 steht;
- m für 0, 2 oder 3 steht;
und Salze, Solvate und Hydrate davon.

2. Verbindung der Formel (I) nach Anspruch 1, worin:
- R₁ für ein Wasserstoffatom oder eine C₁-C₄₋Alkylgruppe steht;
- R₂ für eine NR₉R₁₀-Gruppe oder einen nichtaromatischen carbocyclischen C₃-C₁₂-Rest, der gegebenenfalls ein- oder mehrfach durch eine Methylgruppe substituiert ist, steht;
- R₃, R₄, R₅, R₆, R₇ und R₈ jeweils unabhängig voneinander für ein Wasserstoff- oder Halogenatom, eine C₁-C₆-Alkyl-, C₁-C₆-Alkoxy- oder Trifluormethylgruppe stehen;
- R₉ und R₁₀ gemeinsam mit dem Stickstoffatom, an das sie gebunden sind, einen einen gesättigten oder ungesättigten heterocyclischen Rest mit 5 bis 10 Atomen bilden, der gegebenenfalls ein zweites unter O und N ausgewähltes Heteroatom enthält und gegebenenfalls ein- oder mehrfach durch eine C₁-C₄-Alkylgruppe substituiert ist;
und Salze, Solvate und Hydrate davon.

3. Verbindung der Formel (I) nach Anspruch 1 oder 2, worin:
- R₁ für ein Wasserstoffatom steht und/oder
- R₂ für eine unter Piperidin-1-yl, Pyrrolidin-1-yl, Cyclohexyl, Spiro[5.5]undecanyl, 1,3,3-Trimethylbicyclo[2.2.1]heptan-2-yl steht und/oder
- mindestens einer der Substituenten R₃, R₄ und R₅ für ein Halogenatom oder eine Trifluormethylgruppe steht und/oder
- mindestens einer der Substituenten R₆, R₇ und R₈ für ein Halogenatom steht.

4. Verfahren zur Herstellung einer Verbindung der Formel (I) nach einem der Ansprüche 1 bis 3, **dadurch gekennzeichnet, daß** man ein funktionelles Derivat von Terphenylsäure der Formel: 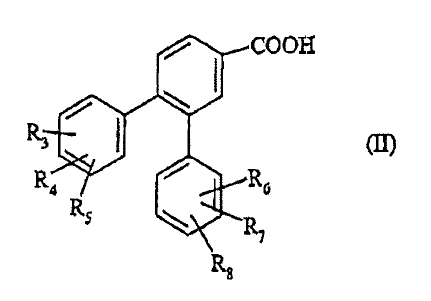 worin R₃, R₄, R₅, R₆, R₇ und R₈ die für eine Verbindung der Formel (I) in Anspruch 1 definierte Bedeutung besitzen, mit einem Amin der Formel HNR₁R₂ (III), worin R₁ und R₂ die für eine Verbindung der Formel (I) in Anspruch 1 definierte Bedeutung besitzen, umsetzt.

5. Verbindungen der Formel: 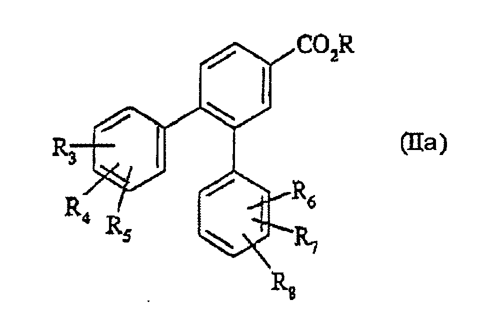 worin R₃, R₄, R₅, R₆, R₇ und R₈ die für eine Verbindung der Formel (I) in Anspruch 1 definierte Bedeutung besitzen und R für ein Wasserstoffatom oder eine C₁-C₄-Alkylgruppe steht, mit der Maßgabe, daß R₃, R₄, R₅, R₆, R₇ und R₈ nicht gleichzeitig für Wasserstoff stehen, und mit der Maßgabe, daß dann, wenn R₄, R₅, R₇ und R₈ für Wasserstoff stehen, R₃ und R₆ nicht gleichzeitig für ein Fluoratom in meta-Stellung oder eine Methoxygruppe in meta- oder para-Stellung stehen, und mit der Maßgabe, daß dann, wenn R₅ und R₈ für Wasserstoff stehen, R₃, R₄ und R₅, R₆ nicht gleichzeitig für 3,4-Dimethoxygruppen stehen.

6. Verbindung der Formel (IIa) nach Anspruch 5, worin:
- R₃ in 4-Stellung steht und für ein Halogenatom oder eine Trifluormethylgruppe steht;
- R₆ in 2-Stellung steht und für ein Wasserstoff- oder Halogenatom steht;
- R₇ in 4-Stellung steht und für ein Halogenatom steht;
- R₄, R₅ und R₈ für Wasserstoff stehen.

7. Medikament, **dadurch gekennzeichnet, daß** es eine Verbindung der Formel (I) nach einem der Ansprüche 1 bis 3 oder ein pharmazeutisch unbedenkliches Salz, Hydrat oder Solvat davon enthält.

8. Pharmazeutische Zusammensetzung, **dadurch gekennzeichnet, daß** sie eine Verbindung der Formel (I) nach einem der Ansprüche 1 bis 3 oder ein pharmazeutisch unbedenkliches Salz, Hydrat oder Solvat davon sowie mindestens einen pharmazeutisch unbedenklichen Hilfsstoff enthält.

9. Verwendung einer Verbindung der Formel (I) nach einem der Ansprüche 1 bis 3 zur Herstellung eines Medikaments für die Behandlung von Erkrankungen, an denen der CB₁-Cannabinoidrezeptor beteiligt ist.

10. Verwendung einer Verbindung der Formel (I) nach einem der Ansprüche 1 bis 3 zur Herstellung eines Medikaments für die Behandlung von psychotischen Störungen, Gedächtnisstörungen, kognitiven Störungen, Appetitstörungen und Adipositas oder für die Tabak- oder Alkoholentwöhnung.
